# EUROPÄISCHE PATENTSCHRIFT

(11) **EP 1 992 323 B1**
(45) Veröffentlichungstag und Bekanntmachung des Hinweises auf die Patenterteilung: **08.02.2012**
(21) Anmeldenummer: 07107950.3
(22) Anmeldetag: 10.05.2007
(51) Int. Cl.: A61K 8/04, A61K 8/27, A61K 8/29, A61Q 19/00, A61K 8/06

(54) **Tensidfreie Schaumformulierungen**
Tenside-free foam formulae
Formulations mousseuses sans tensioactif

(43) Veröffentlichungstag der Anmeldung: 19.11.2008
(73) Patentinhaber: Neubourg Skin Care GmbH & Co. KG, 48268 Greven (DE)
(72) Erfinder: Daniels, Rolf, 72108 Rottenburg a. N. (DE)
(74) Vertreter: Ehlich, Eva Susanne

(56) Entgegenhaltungen:
- WO-A-03/049715
- WO-A-2004/060063
- WO-A1-2004/017930
- DE-A1- 10 162 840

## Beschreibung

### Technisches Gebiet der Erfindung

Die vorliegende Erfindung betrifft kosmetische und dermatologische Schaumformulierungen erhältlich durch Versprühen von feststoffstabilisierten Emulsionen vom Typ Öl-in-Wasser, die frei sind oder im Wesentlichen frei sind von herkömmlichen Emulgatoren. Insbesondere betrifft die Erfindung die Verwendung solcher Pickering-Emulsionen für die Herstellung von Schäumen.

### Hintergrund der Erfindung

### 1. Emulsionen:

Unter Emulsion versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z.B. Milch). Der Grundcharakter einer O/W-Emulsion ist durch das Wasser geprägt. Bei einer Wasser-in-Öl-Emulsion (W/O-Emulsion, z.B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (liphophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilien Molekülteil in ionische (anionische, kationische und amphotere) und nichtionische untergliedert werden:
- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären AmmoniumVerbindungen.
- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man z. B. Hautpflegeemulsionen, so können polare Ölkomponenten wie z. B. UV-Filter zu Instabilitäten führen. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar, welcher in den unterschiedlichsten Anwendungsgebieten eingesetzt wird. So stehen für die Pflege der Haut, insbesondere für die Rückfettung trockener Haut, eine Vielzahl von Produkten - wie Lotionen und Cremes - zur Verfügung. Ziel der Hautpflege ist es, den durch tägliches Waschen verursachten Fett- und Wasserverlust der Haut auszugleichen. Außerdem sollen Hautpflegeprodukte vor Umwelteinflüssen - insbesondere vor Sonne und Wind - schützen und die Hautalterung verzögern.

Kosmetische Emulsionen werden auch als Desodorantien verwendet. Solche Formulierungen dienen dazu, Körpergeruch zu beseitigen, der entsteht, wenn der an sich geruchlose frische Schweiß durch Mikroorganismen zersetzt wird.

Auch zur Reinigung der Haut und Hautanhangsgebilde finden Emulsionen in Form von Reinigungsemulsionen Verwendung. Sie werden meist der Gesichtsreinigung und insbesondere zur Entfernung von dekorativer Kosmetik eingesetzt. Derartige Reinigungsemulsionen haben - im Gegensatz zu anderen Reinigungszubereitungen wie beispielsweise Seife - den Vorteil, besonders hautverträglich zu sein, da sie in ihrer lipophilen Phase pflegende Öle und/oder unpolare Wirkstoffe - wie z.B. Vitamin E - enthalten können.

### 2. Emulgatorfreie Emulsionen

Eine besondere Form der Emulsion stellen emulgatorfreie Emulsionen dar. Diese sind frei von Emulgatoren im engeren Sinne, d.h. von amphiphilen Substanzen mit niedriger Molmasse (Molmasse < 5000), die in höherer Konzentration Mizellen und/oder andere flüssigkristalline Aggregate bilden können.

### 3. Pickering-Emulsion

Eine besondere Form der emulgatorfreien Emulsion stellt die Pickering-Emulsion dar. Pickering-/feststoffstabilisierte Emulsionen sind durch feinstverteilte Feststoffteilchen stabilisiert und ermöglichen es, auf herkömmliche Emulgatoren weitestgehend zu verzichten.

In Pickering-Emulsionen kommt es zu einer Anreicherung des festen Stoffes an der Phasengrenze Öl/Wasser in Form einer Schicht, wodurch ein Zusammenfließen der dispersen Phasen verhindert wird. Von wesentlicher Bedeutung sind dabei insbesondere die Benetzungseigenschaften der Feststoffpartikel, welche sowohl von hydrophilen als auch lipophilen Phasen benetzt werden sollten.

### 4. Schaumformulierungen:

Eine besondere Anwendungsform kosmetischer und oder dermatologischer Emulsionen ist die Applikation als Schäume. Schaumformulierungen haben den Vorteil, sich leicht auf der Haut verteilen zu lassen. Die schaumige Konsistenz wird als angenehm empfunden und die Produkte hinterlassen in der Regel ein gutes Hautgefühl. Insbesondere wirkt sich aber auch die physikalische Struktur des Schaumes positiv auf die Hautschutzfunktion aus. Schäume sind komplizierte physikalische Gebilde, die einer besonderen Abstimmung der den Schaum bildenden Komponenten bedürfen. Schäume werden generell durch Versprühen einer Emulsionsformulierung oder einer wässrigen Tensid-(Stabilisator-)lösung erhalten. Beispielsweise wird die mit Treibgas versetzte Emulsion aus einem unter Druck stehenden Behältnis abgegeben (solche Systeme werden in der Literatur und Patentliteratur auch als Aerosolschäume bezeichnet). Dabei expandiert das unter Druck stehende Gemisch aus Emulsion und Treibgas und bildet die Schaumbläschen. Insbesondere kommt es zu einer Expansion der dispersen Ölphase, in welcher das öllösliche Gas gelöst ist. Schäume können aber auch mit Hilfe anderer Systeme erzeugt werden, wie beispielsweise Pumpsprays.

Abgestimmte Schaumformulierungen weisen eine stabile zwei- oder mehrphasige polydisperse Struktur bei der Applikation auf, die auf der Haut eine Netzstruktur, vergleichbar mit einer Membran bildet. Solche Netzstrukturen haben den Vorteil, dass diese eine Schutzfunktion, beispielsweise vor Kontakt mit Wasser ausbilden, jedoch einen ungehinderten Gasaustausch mit der Umgebung zulassen. Bei solchen Schäumen kommt es praktisch zu keiner Behinderung der Perspiratio insensibiles und keinem entsprechenden Wärmestau. Damit werden die positiven Eigenschaften einer Schutz- und Pflegefunktion mit einer unveränderten Hautatmung verbunden.

Bisher bekannte Schaumformulierungen enthalten herkömmliche Tenside/Emulgatoren, die für die Stabilisierung der Emulsion und für die darauf folgende Schaumstabilität sorgen.

Herkömmliche Emulgatoren bzw. Tenside werden jedoch immer wieder als Ursache für Unverträglichkeiten bei Hautpflegeprodukten genannt, wie z.B. eine Störung der Hautbarriere oder Mallorcaakne. Auf den Zusatz geeigneter Stabilisatoren kann allerdings nicht gänzlich verzichtet werden, da disperse Systeme, wie bereits beschrieben, beispielsweise Emulsionen thermodynamisch instabil sind.

Die oben genannten Pickering-Emulsionen sind eine Möglichkeit, herkömmliche Emulgatoren zu vermeiden. In der EP 1 352 639 A1 bzw. der DE 101 62 840 werden Pickering-Emulsionen vorgestellt, die jedoch als Emulsionen in Form von Lotionen, Cremes und Gelen zum Einsatz kommen.

In der WO 2004/017930 sind weitere Pickering-Emulsionen beschrieben, die sich insbesondere durch eine niedrige Viskosität auszeichnen und damit für dermatologische Tücher geeignet sind. Solche dünnflüssigen Pickering-Emulsionen lassen sich sogar unter einer Nebelbildung versprühen.

Keines der oben beschriebenen Dokumente beschreibt jedoch Schaumformulierungen auf Basis von emulgatorfreien Pickering-Emulsionen.

### Zusammenfassung der Erfindung:

Die Anmelderin hat nun erkannt, dass sich Pickering-Emulsionen als Basis für Schaumformulierungen eignen. Dabei werden die positiven Eigenschaften der Schaumformulierungen mit denen der Pickering-Emulsionen verbunden. Dabei lassen sich insbesondere Schaumformulierungen ohne herkömmliche Emulgatoren oder mit sehr geringen Anteilen an herkömmlichen Emulgatoren herstellen, die die positiven Eigenschaften des Schaumes, nämlich die physikalische Struktur und angenehme Anwendbarkeit, mit der guten Hautverträglichkeit verbinden. Diese Kombination der Eigenschaften macht solche Schaumformulierung insbesondere verwendbar für kosmetische und dermatologische Formulierungen für empfindliche Hauttypen. Es wird dabei Verträglichkeit und Anwendungskomfort vorteilhaft miteinander verbunden.

Dabei ist zunächst davon auszugehen, dass es nicht selbstverständlich ist, dass das Verschäumen von Feststoff-stabilisierten Pickering-Emulsionen zu stabilen Schaumprodukten führt. Schäume werden, wie bereits erwähnt, beispielsweise durch Einarbeiten von Treibgasen in O/W-Emulsionssystemen erhalten. Verdampft bei dem Verschäumen das in der dispersen Ölphase gelöste Treibgas, bildet sich ein Schaum (Gas in Flüssigdispersion). Beim Verdampfen bzw. Expandieren des in der dispersen Ölphase gelösten Treibgases kommt es zu einer Dilatation der dispersen Ölphase. Es ist nun überraschend, dass das Feststoffnetzwerk an der Phasengrenzfläche dieser Dehnungsbelastung stand hält und es beim Verschäumen nicht zum Brechen der Zubereitung kommt.

Die Erfindung betrifft somit Schaumformulierungen gemäß Anspruch 1.

Ferner betrifft die Erfindung die Verwendung von anspruchsgemäßen Emulsionen, insbesondere Pickeringemulsionen, die frei von herkömmlichen Emulgatoren sind, für die Herstellung von Schaumformulierungen.

Ferner betrifft die Erfindung die Verwendung von Schaumformulierungen auf Basis von Pickeringemulsionen als Träger für Wirkstoffe, als Hautpflegemittel, als Hautreinigungsmittel oder als Sonnenschutzmittel. Die Schaumformulierung kann daher als Kosmetikum, Medizinprodukt oder Arzneimittel eingesetzt werden.

Außerdem umfasst die Erfindung ein Verfahren zur Herstellung von Schaumformulierungen basierend auf Pickeringemulsionen. Das Verfahren umfasst die Schritte:
a) Herstellen einer Pickeringemulsion vom Typ Öl in Wasser
b) Abfüllen der Pickeringemulsion und Treibgas in einen Druckbehälter, oder
c) Abfüllen der Pickeringemulsion in einen anderen als einen Druckbehälter, der bei Abgabe der Pickeringemulsion einen Schaum erzeugt

### Detaillierte Beschreibung der Erfindung:

### 1. Definitionen:

Gemäß der vorliegenden Erfindung sind Schaumformulierungen enthältlich durch Versprühen von feststoffstabilisierten Emulsionen, die zur Erzeugung von Schaum sinnfällig hergerichtet sind. Die Formulierungen können insbesondere entweder mit Treibgas in einen Druckbehälter abgefüllt sein oder ohne Treibgas in einen anderen Behälter als einen Druckbehälter abgefüllt sein, der es ermöglicht, bei Abgabe der Formulierung/Emulsion einen Schaum zu erzeugen. Pumpspraybehälter können beispielsweise verwendet werden.

Gemäß der vorliegenden Erfindung sind im Wesentlichen emulgatorfreie Emulsionen solche Emulsionen, die nicht mehr als 0,5 Gew.-% herkömmlicher Emulgatoren enthalten. Gemäß der Erfindung sind emuglatorfreie Emulsionen solche, die keine herkömmlichen Emulgatoren enthalten.

Gemäß der vorliegenden Erfindung ist eine Pickeringemulsion eine feststoffstabilisierte Emulsion. Insbesondere handelt es sich gemäß der Erfindung um eine feststoffstabilisierte Emulsion vom Typ Öl in Wasser (O/W Emulsion). Insbesondere umfasst eine feststoffstabilisierte Emulsion gemäß der Erfindung nicht mehr als 0,5 Gew.-% herkömmliche Emulgatoren. Bevorzugt sind die Pickeringemulsionenen gemäß der Erfindung frei von herkömmlichen Emulgatoren.

Gemäß einem Aspekt sind herkömmliche Emulgatoren gemäß der vorliegenden Erfindung anionische, kationische, amphotere und nichtionische Tenside. Typische Vertreter der anionischen Tenside sind neutralisierte verzweigte und/oder unverzweigte, gesättigte oder ungesättigte Fettsäuren mit einer Kettenlänge von 10 bis 40 Kohlenstoffatomen. Typische Vertreter der kationischen Tenside sind Ammoniumverbindungen. Typische Vertreter der nicht ionischen Tenside haben einen hydrophilen Molekülteil, wie etwa Glycerin, Polyglycerin, Sorbitan, Kohlenhydrate bzw. Polyoxyethlenglykole, der über Ester- und/oder Etherbindungen mit dem lipophilen Molekülteil verknüpft ist, der üblicherweise aus Fettalkoholen, Fettsäuren oder Isofettsäuren besteht. Zum Beispiel zählen die polyethoxylierte Fettsäureester mit Kettenlängen von 10 bis 40 Kohlenstoffatomen und mit einem Ethoxylierungsgrad von 5 bis 100 zu dieser Gruppe. Ferner gelten gesättigte und/oder ungesättigte, verzweigte und/oder unverzweigte Fettalkohole mit einer Kettenlänge von 10 bis 40 Kohlenstoffatomen zu den nichtionischen Emulgatoren. Häufig werden herkömmliche Emulgatoren in Kombinationen eingesetzt. Gemäß einem weiteren Aspekt der Erfindung sind herkömmliche Emulgatoren alle amphiphile Substanzen mit einer Molmasse < 5000, die in höherer Konzentration Mizellen und/oder andere Flüssigkristalline Aggregate bilden können, oder gemäß noch einem weiteren Aspekt sind herkömmliche Emulgatoren alle oberflächenaktiven Substanzen, die insbesondere nicht als Feststoff oder Polymer vorliegen.

Gemäß der Erfindung ist ein Feststoffemulgator eine partikuläre Substanz, die sowohl von lipophilen als auch von hydrophilen Flüssigkeiten benetzbar ist. Es kann sich hierbei um anorganische oder organische Feststoffe handeln. Die Partikel können ferner unbehandelt oder beschichtet sein. Bevorzugt liegt die Partikelgröße zwischen 1 nm und 200 nm, mehr bevorzugt zwischen 5 nm und 100 nm.

### 2. Zusammensetzung der Pickeringemulsionen

### Feststoffemulgatoren:

Geeignete Feststoffemulgatoren sind partikuläre anorganische oder organische Feststoffe, die sowohl von lipophilen als auch von hydrophilen Flüssigkeiten benetzbar sind. Geeignete Vertreter sind z.B. Titandioxid, insbesondere beschichtetes Titanoxid (z.B. erhältlich von Merck KGaA unter der Bezeichnung Eusolex® T-2000), Zinkoxid (z.B. erhältlich von BASF AG unter der Bezeichnung Z-Cote Max) Siliziumdioxid, insbesondere hochdisperses Siliziumdioxid, Fe₂O₃, Veegum, Bentonit und Ethylcellulose. Ferner können Aluminiumoxid, Kohle, Magnesiumoxid, Magnesiumtrisilikat, kristalline Fettalkohole und Fettsäuren, Polymerlatices, z. B. Polystyrole oder Polymethacrylate, und Polymer-Pseudolatices verwendet werden. Auch Mischungen der vorgenannten Feststoffemulgatoren können verwendet werden. Beschichtetes Titandioxid oder Zinkoxid werden bevorzugt.

Die erfindungsgemäßen Emulsionen enthalten mehr als 1 Gew.-% Feststoffemulgator, bevorzugt mehr als 2 Gew.-% Feststoffemulgator, insbesondere von 2 bis 7 Gew.-% Feststoffemulgator. Besonders bevorzugt enthalten die erfindungsgemäßen Emulsionen 3 bis 4 Gew.-% Feststoffemulgator. Die Angaben werden jeweils auf das Gesamtgewicht der Emulsion ohne Treibgas bezogen.

### Ölphase:

Geeignete Komponenten, welche die Ölphase bilden können, können aus den polaren und unpolaren Ölen oder deren Mischungen gewählt werden.

Die Ölphase der erfindungsgemäßen Formulierungen wird vorteilhaft gewählt aus der Gruppe der Lecithine und der Fettsäuretriglyceride, aus der Gruppe der Propylenglykole oder Butylenglykole, Fettsäureester, aus der Gruppe der natürlichen Wachse, tierischen und pflanzlichen Ursprungs, aus der Gruppe der Esteröle, aus der Gruppe der Dialkylether und Dialkylcarbonate, aus der Gruppe der verzweigten und unverzweigten Kohlenwasserstoffe und Wachse sowie aus der Gruppe der zyklischen und linearen Silikonöle.

Besonders bevorzugt sind Triglyceride insbesondere Caprylsäure-/Caprinsäuretriglycerid erhältlich unter der Bezeichnung Miglyol 812 der Firma Sasol und dessen Abmischung mit weiteren Öl- und Wachskomponenten.

Besonders bevorzugt sind Triglyceride, insbesondere Caprylsäure-/Caprinsäuretriglycerid, erhältlich unter der Bezeichnung Miglyol 812 der Firma Sasol /Myritol 312 der Fa. Cognis.

Die erfindungsgemäßen Emulsionen enthalten bevorzugt von 10 bis 50 Gew.-% Ölphase, besonders bevorzugt 25 bis 35 Gew.-% Ölphase. Die Angaben werden jeweils auf das Gesamtgewicht der Emulsion ohne Treibgas bezogen.

### Wasserphase:

Die Wasserphase kann kosmetische Hilfsstoffe enthalten, z.B. niedere Alkohole (z.B. Ethanol, Isopropanol), niedere Diole oder Polyole sowie deren Ether (z.B. Propylenglycol, Glycerin, Butylenglykol, Hexylenglykol und Ethylenglykol), Schaumstabilisatoren und Verdickungsmittel.

Geeignete Verdickungsmittel sind polymere Verdickungsmittel, die teilweise wasserlöslich oder zumindest in Wasser disperierbar sind und in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie die Beweglichkeit des Wassers einschränken. Geeignete Polymere sind:
- Abgewandelte Naturstoffe, wie Celluloseether (z.B. Hydroxypropylcelluloseether, Hydroxyethlylcellulose und Hydroxypropylmethylcelluloseether);
- Natürliche Verbindungen, wie z.B. Agar-Agar, Carrageen, Polyosen, Stärke, Dextrine, Gelatine, Casein;
- Synthetische Verbindungen wie z.B. Vinylpolymere, Polyether, Polyimine, Polyamide und Derivate der Polyacrylsäure; und
- Anorganische Verbindungen wie z.B. Polykieselsäure und Tonmineralien.

Bevorzugt ist ein Celluloseether enthalten. Besonderes bevorzugt ist Hydroxypropylmethylcellulose.

Die erfindungsgemäßen Emulsionen enthalten bevorzugt von 0,2 bis 1,5 Gew.-% Verdickungsmittel (bezogen auf das Trockengewicht des Verdickungsmittels und das Gesamtgewicht der Emulsion ohne Treibgas. Besonders bevorzugt sind 0,5 bis 0,8 Gew.-% Verdickungsmittel.

### Wirkstoffe:

Der enthaltene Wirkstoff kann unter allen oberflächig auf der Haut applizierbaren Wirkstoffen und Mischungen dieser gewählt werden. Der Wirkstoff kann kosmetisch oder pharmazeutisch wirken. Entsprechend erhält man kosmetische oder dermatologische (als Medizinprodukt oder Arzneimittelprodukt einzusetzende) Schaumformulierungen. Ferner kann die Formulierung zum Schutz der Haut vor Umwelteinflüssen dienen. Der Wirkstoff kann rein pflanzlich oder synthetisch sein. Die Gruppe der Wirkstoffe kann sich auch mit den anderen Inhaltstoffgruppen, wie z.B.der Ölkomponente, den Verdickungsmitteln oder den Feststoffemulgatoren, überschneiden. Beispielsweise können manche Ölkomponenten auch als Wirkstoffe dienen, wie z.B. Öle mit mehrfach ungesättigten Fettsäuren, oder Feststoffemulgatoren, wie z.B. partikuläres Titandioxid als UV-Filter dienen kann. Je nach Eigenschaftsprofil sind die Substanzen mehreren Gruppen zuzuordnen.

Wirkstoffe der erfindungsgemäßen Formulierungen werden vorteilhaft gewählt aus der Gruppe der Substanzen mit feuchtigkeitspendenden und barrierestärkenden Eigenschaften, wie z. B. Hydroviton, eine Nachbildung des NMF, Pyrrolidoncarbonsäure und deren Salze, Milchsäure und deren Salze, Glycerol, Sorbitol, Propylenglykol und Harnstoff, Substanzen aus der Gruppe der Proteine und Proteinhydrolysate wie z. B. Collagen, Elastin sowie Seidenprotein, Substanzen aus der Gruppe der Glucosaminoglucane, wie z. B. Hyaluronsäure, aus der Gruppe der Kohlenhydrate, wie z. B. Pentavitin, das in seiner Zusammensetzung dem Kohlehydratgemisch der menschlichen Hornschicht entspricht, und der Gruppe der Lipide und Lipidvorstufen wie beispielsweise die Ceramide. Weitere vorteilhafte Wirkstoffe können im Sinne der vorliegenden Erfindung ferner gewählt werden aus der Gruppe der Vitamine, wie z. B. Panthenol, Niacin, α-Tocopherol und seine Ester, Vitamin A sowie Vitamin C. Außerdem können die Wirkstoffe, gewählt aus der Gruppe der Antioxidantien z. B. Galate und Polyphenole, verwendet werden. Harnstoff, Hyaluronsäure und Pentavitin sind bevorzugte Substanzen.

Es ist ferner bevorzugt, dass als Wirkstoffe Substanzen mit hautberuhigender und regenerierender Wirkung eingesetzt werden, wie z. B. Panthenol, Bisabolol und Phytosterole.

Vorteilhafte Wirkstoffe im Sinne der vorliegenden Erfindung sind auch Pflanzen und Pflanzenextrakte. Hierzu gehören zB Algen, Aloe, Arnika, Bartflechten, Beinwell, Birke, Brennessel, Calendula, Eiche, Efeu, Hamamelis, Henna, Hopfen, Kamille, Mäusedorn, Pfefferminze, Ringelblume, Rosmarin, Salbei, grüner Tee, Teebaum, Schachtelhalm, Thymian und Walnuss sowie deren Extrakte.

Die erfindungsgemäßen Zubereitungen können ferner als Wirkstoffe Antimykotika und Antiseptika/Desinfizientia synthetischen oder natürlichen Ursprungs enthalten.

Weitere Wirkstoffe sind Glucocortikoide, Antibiotika, Analgetika, Antiphlogistika, Antirheumatika, Antiallergika, Antiparasitika, Antipruriginosa, Antipsoriatika, Retinoide, Lokalanästhetika, Venentherapeutika, Keratolytika, Hyperämisierende Substanzen, Koronartherapeutika (Nitrate/Nitro-Verbindungen), Virusstatika, Zytostatika, Hormone, Wundheilungsfördernde Wirkstoffe, zB Wachstumsfaktoren, Enzympräparate und Insektizide.

### Weitere Bestandteile der Pickeringemulsion:

Die Formulierungen können Farbstoffe, Perlglanzpigmente, Duftstoffe/Parfum, Lichtschutzfiltersubstanzen, Konservierungsmittel, Komplexbildner, Antioxidantien und Repellentien enthalten.

Die obige Aufzählung der einzelnen Bestandteile der Pickeringemulsion soll so verstanden werden, dass einzelne Beispielkomponenten aufgrund ihrer verschiedenen Eigenschaften auch mehreren Gruppen zugeordnet werden können.

### Treibgase:

Geeignete Treibgase sind z. B. N₂O, Propan, Butan und i-Butan, Die fertige Schaumformulierung enthält von 5 bis 15 Gew.-%, bevorzug etwa 10 Gew.-% Treibgas.

### 3. Herstellungsverfahren:

Die Schaumformulierungen gemäß der Erfindung werden durch Bereitstellen einer Emulsion bzw. Pickeringemulsion vom Typ Öl in Wasser und Abfüllen der Emulsion bzw. Pickeringemulsion und gegebenenfalls Treibgas in einen gegebenenfalls Druckbehälter hergestellt. Alternativ zum Treibgas und Druckbehälter kann die Pickeringemulsion auch in einen anderen Behälter abgefüllt werden, der auch ohne Treibgas dafür geeignet ist, die Pickeringemulsion als Schaum abzugeben.

Insbesondere wird die Pickeringemulsion in folgenden Schritten hergestellt:
1. Bereitstellen einer liquiden Ölphase
2. Suspendieren der oder des Feststoffemulgators in der Ölphase, um eine Feststoffemulgatorsuspension zu erhalten
3. Bereitstellen einer Wasserphase
4. Homogenisieren der Wasserphase mit der Feststoffemulgatorsuspension, um eine Pickeringemulsion zu erhalten.

Bevorzugt wird die Ölphase und die Wasserphase bei einer Temperatur von 50 bis 90 °C, bevorzugt von 50 bis 70 °C und besonders bevorzugt bei ca. 60°C homogenisiert.

Wenn die Pickeringemulsion ein Verdickungsmittel umfasst, kommen vorteilhaft ferner die folgenden Schritte hinzu:
5. Bereitstellen einer wässrigen Verdickungsmittellösung
6. Mischen der Verdickungsmittellösung mit der Pickeringemulsion.

Bevorzugt wird die Pickeringemulsion mit 10 Gew.- Treibgas zur Herstellung der Schaumformulierung beaufschlagt.

### 4. Verwendungen:

Die Schaumformulierungen der vorliegenden Erfindung können für alle kosmetischen und dermatologischen (als Medizinprodukt oder Arzneimittel) Zwecke eingesetzt werden. Zum Beispiel könne die Formulierungen als Hautpflegemittel oder Hautreinigungsmittel eingesetzt werden. Sie könne ferner als Träger für Wirkstoffe dienen und im medizinisch dermatologischen Bereich eingesetzt werden. Insbesondere können die Formulierungen als Sonnenschutzmittel eingesetzt werden. Viele der Feststoffemulgatoren, wie beispielsweise Titandioxid, sind wirksame UVA und UVB Filter.

### 5. Beispiel:

Zusammensetzung der primären Pickeringemulsion:
10,00 g Eusolex T 2000 der Firma Merck KGaA (Alumina/Simethicone beschichtetes Titandioxid)
95,00 g Miglyol 812 der Firma Sasol (Caprylsäure/Caprinsäuretriglycerid)
95,00 g Wasser

Zusammensetzung der Schaumformulierung:
60,00 g primäre Pickeringemulsion
30,00 g wässrige Hypromelloselösung (2 %)
10,00 g Treibgas

### Herstellung der primären Pickeringemulsion:

Eusolex T2000 wird abgewogen und in einen Laborhomogenisator überführt und mit dem auf 60°C erwärmten Miglyol 812 innerhalb von einer Minute gemischt sowie innerhalb von 5 Minuten homogenisiert. Das Wasser wird auf 60°C erwärmt und bei 1000 U/min innerhalb von einer Minute zugegeben. Die Mischung wird bei 3000 U/Min für 5 Minuten homogenisiert.

### Herstellung der Schaumformulierung:

30,00 g Hypromellose-Lösung werden zu 60,00 g der primären Pickeringemulsion unter Rühren zugegeben. Die hypromellosehaltige Pickeringemulsion wird dann in Aluminiummonoblockdosen mit 10,00 g Treibgas beaufschlagt.

## Patentansprüche

1. Kosmetische oder dermatologische Schaumformulierung, erhaltlich durch Versprühen einer feststoffstabilisierten Emulsion vom Typ Öl in Wasser, umfassend eine Ölphase und eine Wasserphase, wobei die Emulsion nicht mehr als 0,5 Gew.-% herkömmlicher Emulgatoren enthält, wobei ein herkömmlicher Emulgator eine Substanz ist mit einem Molekülaufbau, bestehend aus einem hydrophilen und einem lipophilen Molekülteil, die räumlich voneinander getrennt sind, wobei die Substanz an der Phasengrenze Öl/Wasser Grenzflächenfilme ausbildet und eine Molmasse <5000 aufweist.

2. Schaumformulierung gemäß Anspruch 1, umfassend eine emulgatorfreie Emulsion, die keine herkömmlichen Emulgatoren enthält.

3. Schaumformulierung gemäß Anspruch 1 oder 2, wobei die Emulsion eine Pickeringemulsion ist.

4. Schaumformulierung gemäß Anspruch 3,
wobei die Pickeringemulsion mindestens einen partikulären Feststoffemulgator, ausgewählt aus der Gruppe, bestehend aus Titandioxid, Siliziumdioxid, Fe₂O₃, Zinkoxid, Veegum, Bentonit und Ethylcellulose, Aluminiumoxid, Kohle, Magnesiumoxid, Magnesiumtrisilikat, kristalline Fettalkohole und Fettsäuren, Polymerlatices, wie etwa Polystyrole oder Polymethacrylate, und Polymer-Pseudolatices oder Mischungen davon umfasst.

5. Schaumformulierung gemäß Anspruch 3,
wobei die Pickeringemulsion beschichtetes Titandioxid oder Zinkoxid umfasst.

6. Schaumformulierung gemäß einem der voran stehenden Ansprüche,
wobei die Ölphase mindestens ein Triglycerid umfasst.

7. Schaumformulierung gemäß Anspruch 6,
wobei das Triglycerid Caprylsäure/Caprinsäuretriglycerid ist.

8. Schaumformulierung gemäß einem der voran stehenden Ansprüche,
wobei die Emulsion mindestens ein Verdickungsmittel umfasst.

9. Schaumformulierung gemäß Anspruch 8,
wobei das Verdickungsmittel Hydroxypropylmethylcellulose ist.

10. Schaumformulierung gemäß einem der voran stehenden Ansprüche,
wobei die Emulsion mindestens einen Wirkstoff enthält.

11. Schaumformulierung gemäß Anspruch 10,
wobei der Wirkstoff aus der Gruppe bestehend aus Hydroviton, Pyrrolidoncarbonsäure und deren Salze, Milchsäure und deren Salze, Glycerol, Sorbitol, Propylenglykol, Harnstoff, Collagen, Elastin, Seidenprotein, Hyaluronsäure, Pentavitin, Ceramide, Panthenol, Niacin, α-Tocopherol und seine Ester, Vitamin A, Vitamin C, Galate, Polyphenole, Panthenol, Bisabolol, Phytosterole, Glucocortikoide, Antibiotika, Analgetika, Antiphlogistika, Antirheumatika, Antiallergika, Antiparasitika, Antipruriginosa, Antipsoriatika, Retinoide, Lokalanästhetika, Venentherapeutika, Keratolytika, Hyperämisierende Substanzen, Koronartherapeutika (Nitrate/NitroVerbindungen), Virusstatika, Zytostatika, Hormone, Wundheilungsfördernde Wirkstoffe, Wachstumsfaktoren, Enzympräparate, Insektizide und Pflanzenmaterial wie, bzw. Pflanzenextrakte von, Algen, Aloe, Arnika, Bartflechten, Beinwell, Birke, Brennnessel, Calendula, Eiche, Efeu, Hamamelis, Henna, Hopfen, Kamille, Mäusedorn, Pfefferminze, Ringelblume, Rosmarin, Salbei, grüner Tee, Teebaum, Schachtelhalm, Thymian und Walnuss oder Mischungen davon, ausgewählt ist.

12. Verwendung einer Pickeringemulsion für die Herstellung einer Schaumformulierung gemäß einem der Ansprüche 1 bis 11.

13. Verwendung einer Schaumformulierung gemäß einem der Ansprüche 1 bis 11 als Träger für einen Wirkstoff.

14. Verwendung einer Schaumformulierung gemäß einem der Ansprüche 1 bis 11 als Hautpflegemittel.

15. Verwendung einer Schaumformulierung gemäß einem der Ansprüche 1 bis 11 als Hautreinigungsmittel.

16. Verwendung einer Schaumformulierung gemäß einem der Ansprüche 1 bis 11 zur Herstellung eines Kosmetikums, Medizinprodukts oder Arzneimittels.

17. Verfahren zur Herstellung einer Schaumformulierung nach einem der Ansprüche 1 bis 11 basierend auf einer Pickeringemulsion, umfassend die Schritte:
a) Herstellen einer Pickeringemulsion vom Typ Öl in Wasser
b) Abfüllen der Pickeringemulsion und Treibgas in einen Druckbehälter, oder
c) Abfüllen der Pickeringemulsion in einen anderen als einen Druckbehälter, der bei Abgabe der Pickeringemulsion einen Schaum erzeugt.

18. Verfahren gemäß Anspruch 17, wobei das Herstellen der Pickeringemulsion die Schritte
1. Bereitstellen einer liquiden Ölphase
2. Suspendieren der oder des Feststoffemulgators in der Ölphase, um eine Feststoffemulgatorsuspension zu erhalten
3. Bereitstellen einer Wasserphase
4. Homogenisieren der Wasserphase mit der Feststoffemulgatorsuspension, um eine Pickeringemulsion zu erhalten,
umfasst.

19. Verfahren gemäß Anspruch 18, wobei die Ölphase und die Wasserphase bei einer Temperatur zwischen 50 und 90 °C homogenisiert werden.

20. Verfahren gemäß Anspruch 18 oder 19, wobei die Pickeringemulsion ein Verdickungsmittel umfasst, ferner umfassend die Schritte:
5. Bereitstellen einer wässrigen Verdickungsmittellösung
6. Mischen der Verdickungsmittellösung mit der Pickeringemulsion.

21. Verfahren gemäß einem der Ansprüche 17 bis 20, wobei die Schaumformulierung 10 Gew.- Treibgas enthält.

## Claims

1. Cosmetic or dermatologic foam formulation obtainable by spraying an emulsion stabilized by solids of the oil-in-water type, comprising an oil phase and a water phase, wherein the emulsion does not contain more than 0.5 wt.-% of conventional emulsifiers, wherein a conventional emulsifier is a substance having a structure consisting of a hydrophilic an a lipophilic part of the molecule, which are separated from each other in space, wherein the substance forms a boundary surface film in the boundary surface between oil phase and water phase and has a molecular weight of < 5000.

2. The foam formulation according to claim 1, comprising an emulsifier-free emulsion that does not contain conventional emulsifiers.

3. The foam formulation according to claim 1 or 2, wherein the emulsion is a Pickering emulsion.

4. The foam formulation according to claim 3, wherein the Pickering emulsion comprises at least one particulate solid emulsifier selected from the group consisting of titanium dioxide, silicon dioxide, Fe₂O₃, zinc oxide, veegum, bentonit and ethyl cellulose, aluminium oxide, coal, magnesium oxide, magnesium trisilicate, crystalline fatty alcohols and fatty acids, polymer lattices such as polystyrene or polymethacrylate, and polymer-pseudolattices or mixtures thereof.

5. The foam formulation according to claim 3, wherein the Pickering emulsion comprises coated titanium dioxide or zinc oxide.

6. The foam formulation according to any one of the preceding claims, wherein the oil phase comprises at least one triglyceride.

7. The foam formulation according to claim 6, wherein the triglyceride comprises caprylic acid/caprinic acid triglyceride.

8. The foam formulation according to any one of the preceding claims, wherein the emulsion comprises at least one thickening agent.

9. The foam formulation according to claim 8, wherein the thickening agent is hydroxypropyl methylcellulose.

10. The foam formulation according to any one of the preceding claims, wherein the emulsion contains at least one active agent.

11. The foam formulation according to claim 10, wherein the active agent is selected from the group consisting of hydroviton, pyrrolidone carbonic acid and salts thereof, lactic acid and salts thereof, glycerol, sorbitol, propylene glycol, urea, collagen, elastin, silk protein, hyaluronic acid, pentavitin, ceramid, panthenol, niacin, α-tocopherol and esters thereof, vitamin A, vitamin C, galates, polyphenols, panthenol, bisabolol, phytosteroles, glycocorticoides, antibiotics, analgetics, antiphlogistics, antirheumatics, antiallergics, antiparasitics, antipruriginosics, antipsoriatics, retinoids, local anaesthetics, venous thereapeutics, ceratolytics, hyperemisic compounds, coronary therapeutics (nitrates/nitro-compounds), virus statics, cytostatics, hormones, agents promoting wound healing, growth factors, enzyme preparations, insecticides and plant material such as plant extracts of algae, aloe, arnica, barber's rash, comfrey, birch, stinging nettle, calendula, oak, ivy, witch hazel, henna, hops, camomile, ruscus, peppermint, marigold, rosemary, sage, green tea, tea tree, horsetail, thyme, and walnut or mixtures thereof.

12. Use of a Pickering emulsion for the manufacture of a foam formulation according t any one of claims 1 to 11.

13. Use of a foam formulation according to any one of claims 1 to 11 as a carrier for an active agent.

14. Use of a foam formulation according to any one of claims 1 to 11 as a skincare agent.

15. Use of a foam formulation according to any one of claims 1 to 11 as a skin cleaning agent.

16. Use of a foam formulation according to any one of claims 1 to 11 for the manufacture of a cosmetic, a medical product or a pharmaceutical composition.

17. A method for producing a foam formulation according to any one of claims 1 to 11 based on a Pickering emulsion comprising the steps:
a) Producing a Pickering emulsion of the oil in water type,
b) Filling the Pickering emulsion and propellant into a pressure vessel, or
c) Filling the Pickering emulsion into a different container as a pressure vessel that produces a foam upon dispensing of the Pickering emulsion.

18. The method according to claim 17, wherein producing of the Pickering emulsion comprises the steps:
2. Suspending one or more solid emulsifiers in the oil phase in order to obtain a suspension of solid emulsifier,
3. Providing an aqueous phase,
4. Homogenizing the aqueous phase with the suspension of solid emulsifier in order to obtain a Pickering emulsion.

19. The method according to claim 18, wherein the oil phase and the aqueous phase are homogenized at a temperature between 50 and 90°C.

20. The method according to claim 18 or 19, wherein the Pickering emulsion comprises a thickening agent, further comprising the steps:
5. Providing an aqueous solution of thickening agent,
6. Mixing the solution of thickening agent with the Pickering emulsion.

21. The method according to one of claims 17 to 20, wherein the foam formulation contains 10 weight percent of propellant.

## Revendications

1. Formulation de mousse cosmétique ou dermatologique, obtenue par pulvérisation d'une émulsion du type huile dans l'eau stabilisée par des solides, renfermant une phase huileuse et une phase aqueuse, l'émulsion ne contenant pas plus de 0,5% en poids d'émulsifiants conventionnels, un émulsifiant conventionnel étant une substance avec une structure moléculaire constituée d'une partie de molécule hydrophile et d'une part de molécule lipophile, séparées dans l'espace l'une de l'autre, la substance formant à la limite de phase huile/eau des films d'interface et présentant une masse molaire < 5000.

2. Formulation de mousse selon la revendication 1, renfermant une émulsion sans émulsifiant, qui ne contient aucun émulsifiant conventionnel.

3. Formulation de mousse selon l'une des revendications 1 ou 2, dans laquelle l'émulsion est une émulsion de Pickering.

4. Formulation de mousse selon la revendication 3, dans laquelle l'émulsion de Pickering comprend au moins un émulsifiant solide particulaire, sélectionné parmi le groupe constitué de dioxyde de titane, de dioxyde de silicium, Fe₂O₃, oxyde de zinc, Veegum, bentonite et éthylcellulose, oxyde d'aluminium, charbon, oxyde de magnésium, trisilicate de magnésium, alcools gras cristallins et acides gras, latices de polymères, tels que par exemple polystyrols ou polyméthacrylates, et pseudo-latices de polymères ou des mélanges de ces derniers.

5. Formulation de mousse selon la revendication 3, dans laquelle l'émulsion de Pickering renferme du dioxyde de titane ou de l'oxyde de zinc enrobé.

6. Formulation de mousse selon l'une des revendications précédentes, dans laquelle la phase huileuse renferme au moins un triglycéride.

7. Formulation de mousse selon la revendication 6, dans laquelle le triglycéride est un triglycéride d'acide caprique/d'acide caprylique.

8. Formulation de mousse selon l'une des revendications précédentes, dans laquelle l'émulsion renferme au moins un épaississant.

9. Formulation de mousse selon la revendication 8, dans laquelle l'épaississant est de l'hydroxypropyl-méthylcellulose.

10. Formulation de mousse selon l'une des revendications précédentes, dans laquelle l'émulsion renferme au moins une substance active.

11. Formulation de mousse selon la revendication 10, dans laquelle la substance active est sélectionnée dans le groupe constitué d'Hydroviton, d'acide pyrrolidone carboxylique et de ses sels, d'acide lactique et de ses sels, de glycérol, de sorbitol, de propylène glycol, d'urée, de collagène, d'élastine, de protéine de soie, d'acide hyaluronique, de Pentavitine, de céramide, de panthénol, de Niacine, d' α-tocophérol et de ses esters, de vitamine A, de vitamine C, de galate, de polyphénols, panthénol, de bisabolol, de phytostérols, de glucocorticoïdes, d'antibiotiques, d'analgésiques, d'antiphlogistiques, d'antirhumatismaux, d'antiallergi-ques, d'antiparasitaires, d'antiprurigineux, d'anti-psoriatiques, de rétinoïdes, d'anesthésiants locaux, de thérapeutiques veineux, de kératolytiques, de substances antiémétiques, de thérapeutiques coronariens (nitrates/dérivés nitrés), de virostatiques, de cytostatiques, d'hormones, de substances actives cicatrisantes, de facteurs de croissance, de préparations enzymatiques, d'insecticides et matières végétales, telles que, par exemple, extraits végétaux d'algues, aloès, arnica, lichen, consoude, bouleau, ortie, calendula, chêne, lierre, hamamélis, henné, houblon, camomille, fragon, menthe, souci, romarin, sauge, thé vert, théier, prêle, thym et noix ou de mélanges de ces derniers.

12. Utilisation d'une émulsion de Pickering pour la préparation d'une formulation de mousse selon l'une des revendications 1 à 11.

13. Utilisation d'une formulation de mousse selon l'une des revendications 1 à 11 en tant que support pour une substance active.

14. Utilisation d'une formulation de mousse selon l'une des revendications 1 à 11 en tant que produit principal de soins.

15. Utilisation d'une formulation de mousse selon l'une des revendications 1 à 11 en tant que nettoyant pour la peau.

16. Utilisation d'une formulation de mousse selon l'une des revendications 1 à 11 pour la préparation d'un cosmétique, d'un produit pharmaceutique ou d'un médicament.

17. Procédé de préparation d'une formulation de mousse selon l'une des revendications 1 à 11 sur la base d'une émulsion de Pickering, comprenant les étapes :
a) préparation d'une émulsion de Pickering du type huile dans l'eau
b) remplissage de l'émulsion de Pickering et du gaz propulseur dans un récipient sous pression, ou
c) remplissage de l'émulsion de Pickering dans un récipient autre qu'un récipient sous pression, qui génère une mousse lors de la sortie de l'émulsion de Pickering.

18. Procédé selon la revendication 17, dans lequel la préparation de l'émulsion de Pickering comprend les étapes
1. Préparation d'une phase huileuse liquide
2. Suspension des ou de l'émulsifiant solide dans la phase huileuse, pour obtenir une suspension d'émulsifiant solide
3. Préparation d'une phase aqueuse
4. Homogénéisation de la phase aqueuse avec la suspension d'émulsifiant solide, pour obtenir une émulsion de Pickering.

19. Procédé selon la revendication 18, dans lequel la phase huileuse et la phase aqueuse sont homogénéisées à une température comprise entre 50 et 90°C.

20. Procédé selon l'une des revendications 18 ou 19, dans lequel l'émulsion de Pickering renferme un épaississant, comprenant en outre les étapes :
5. Préparation d'une solution aqueuse d'épaississant
6. Mélange de la solution d'épaississant avec l'émulsion de Pickering.

21. Procédé selon l'une des revendications 17 à 20, dans lequel la formulation de mousse renferme 10% en poids de gaz propulseur.
